# EUROPEAN PATENT APPLICATION

(11) **EP 1 640 001 A1**
(43) Date of publication of application: **29.03.2006**
(21) Application number: 04447211.6
(22) Date of filing: 24.09.2004
(51) Int. Cl.: A61K 31/353, A61P 9/00

(54) **Composition against cardiovascular diseases**

(71) Applicant: New Pharma Investments Holding, L-1526 Grand Duché de Luxembourg (LU)
(72) Inventor: Gerard, Eddy, 8620 Nieuwpoort (BE)
(74) Representative: Powis de Tenbossche, Roland

(57) **Abstract**

The use of
- at least one vitamin,
- at least one soy derivative, and
- at least one polyphenol,
in which the weight ratio (vitamins) / (polyphenols + soy derivatives) is comprised between 1:10 and 10:1 as therapeutic active mixture for the preparation of an oral therapeutic formulation for preventing and/or treating cardiovascular diseases.

## Description

### Field of the invention

The invention relates to a composition containing at least one vitamins, and to its therapeutic or pharmaceutical use.

### Prior art

The use of vitamins has been proposed for various purposes, such as growth of children, general condition health, health promoting composition, etc.

Each vitamin has specific functions. If levels of a particular vitamin are inadequate, a deficiency disease results.
(see http://www.nlm.nih.gov/medlineplus/ency/article/002399.htm )

The vitamins comprises :
Vitamin A helps in the formation and maintenance of healthy teeth, skeletal and soft tissue, mucous membranes, and skin. It is also known as retinol because it generates the pigments that are necessary for the working of the retina. It promotes good vision, especially in dim light. Vitamin A may also be required for reproduction and breast-feeding. Beta-carotene is a precursor to vitamin A that has antioxidant properties, helping the body deal with unstable chemicals called free radicals.
Thiamine (B1) helps the body cells convert carbohydrates into energy. It is also essential for the functioning of the heart and for healthy nerve cells, including those in the brain.
Riboflavin (B2) works with the other B vitamins and is important for body growth and red blood cell production. Similar to thiamine, it helps in releasing energy from carbohydrates.
Niacin is a B vitamin that helps maintain healthy skin and nerves. It is also important for the conversion of food to energy and may have cholesterol-lowering effects.
Vitamin B6 is also known as pyridoxine. The more protein a person eats, the more vitamin B6 is required to help the body use the protein. It aids in the formation of red blood cells and in the maintenance of normal brain function. It also assists in the synthesizing of antibodies in the immune system.
Vitamin B12, like the other B vitamins, is important for metabolism. It, too, helps in the formation of red blood cells and in the maintenance of the central nervous system.
Pantothenic acid is essential for the metabolism of food. It is also essential in the synthesis of hormones and cholesterol. Biotin is essential for the metabolism of proteins and carbohydrates, and in the synthesis of hormones and cholesterol. Cholesterol is needed for the functioning of cell membranes, particularly in the brain.
Folate (folic acid) works with vitamin B12 in the production of red blood cells. It is necessary for the synthesis of DNA, which controls heredity as well as tissue growth and cell function. Any woman who may become pregnant should be sure to consume enough folate -- low levels of this substance are associated with devastating birth defects such as spina bifida. Many foods are now fortified with folic acid to help reduce the level of such birth defects.
Vitamin C, also called ascorbic acid, promotes healthy teeth and gums, helps in the absorption of iron, and helps maintain normal connective tissue. It also promotes wound healing and is an antioxidant.
Vitamin D is also known as the "sunshine vitamin," since it is manufactured by the body after being exposed to sunshine. Ten to fifteen minutes of sunshine three times per week is adequate to produce the body's requirement of vitamin D. This vitamin promotes the body's absorption of calcium, which is essential for the normal development and maintenance of healthy teeth and bones. It also helps maintain adequate blood levels of calcium and phosphorus, which are minerals necessary for many functions.
Vitamin E is also known as tocopherol and is an antioxidant. It is also important in the formation of red blood cells and the use of vitamin K.
Vitamin K is known as the clotting vitamin, because without it blood would not coagulate. Some studies indicate that it helps in maintaining strong bones in the elderly.

Recently, the FDA was requested to authorize a health claim that a daily intake of 400µg folic acid, 3mg vitamin B6 and 5 µg vitamin B12 may reduce the risk of vascular disease. However, after review o numeral studies, the FDA concludes that a health claim stating that "Folic cid, Vitamin B6 and vitamin B12 may reduce the risk of vascular disease" is misleading.

It has also been suggested (i.e. the French Paradox) that red wine contains compounds improves cardiovascular diseases. However, red wine has never been accepted by a Health Authority as a pharmaceutical composition as such.

Brown and Hu (2001) suggest that n-3 fatty acids, antioxidant vitamins, folic acid and L-arginine have beneficial effects on endothelial function. However, up to now, Health authority has not accepted these compounds as pharmaceutical active agent or with a recognized therapeutic activity.

It has also been suggested that soy isoflavones are nutriments suitable for reducing cardiovascular diseases (see nutranews - September 2004). Soy isoflavones are up to now not recognized by the Food and Drug Administration as pharmaceutical or therapeutic compound for preventing cardiovascular diseases.

A pharmaceutical composition containing at least two phytochemicals selected from the group consisting of isoflavones, lignans, saponins, sapogenins, catechins and phenolic acids has been proposed in US6391308. As stated by the inventors of US6391308, it is expected that some synergism would arise out of the combination. Said inventors have not seen the key role of vitamins.

It has now been discovered that specific compositions had therapeutic efficiency with respect to cardiovascular diseases, and other diseases or troubles associated to cardiovascular diseases, for example troubles considered as a possible cause of cardiovascular disease.

The therapeutic compositions of the invention are pharmaceutical compositions or therapeutic nutriments or nutriceuticals (pharmaceutical nutriment or nutriment having a recognized pharmaceutical or therapeutic activity).

The invention is based on a synergetic therapeutic activity between vitamins, polyphenols and soy derivatives enabling a recognizable therapeutic efficiency against various possible causes of cardiovascular diseases.

### Brief description of the invention

The invention relates thus to the use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, such as a pharmaceutical formulation or a nutriment formulation with recognized therapeutic efficiency, especially an oral unit dosage therapeutic form, for preventing and/or treating cardiovascular diseases.
The weight ratio in said composition or use is thus the ratio (vitamin or vitamins) / [ (soy derivatives which are not a vitamin) + (polyphenols which are not a vitamin, nor a soy derivative)] is comprised between 1:10 and 10:1, advantageously 1:2 and 5:1.
The vitamin, the soy derivative and the polyphenol used in the present invention are compounds used in an amount which is therapeutically acceptable for human.

More specifically, the invention relates to the use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, :
- for reducing the plasma fibrinogen level and/or
- for reducing the blood CRP (C-reactive protein) level, and/or
- for preventing or treating atherosclerosis, and/or
- for treating or preventing inflammation troubles, preferably vascular inflammation, and/or
- for preventing or treating endothelial dysfunction, and/or
- for preventing or treating diabetic vasculitis, and/or
- for preventing or treating metabolic syndrome and/or
- for controlling the human CRP gene expression and/or
- for preventing or treating hypertension and/or
- for preventing or treating troubles associated to unstable angina pectoris and/or
- for controlling or reducing the formation of triglycerides and/or
- for controlling the ratio HDL/LDL and/or reducing the LDL content.
The weight ratio in said composition or use is thus the ratio (vitamin or vitamins) / [ (soy derivatives which are not a vitamin) + (polyphenols which are not a vitamin, nor a soy derivative)] is comprised between 1:10 and 10:1, advantageously 1:2 and 5:1.

The composition of the invention used for said purposes comprises at least three different compounds, at least a vitamin, at least a soy derivative which is not a vitamin, and at least a polyphenol which is not a vitamin, nor a soy derivative.

Advantageously, in said use, the oral therapeutic formulation comprises at least vitamin B12 and at least another vitamin selected from the group consisting of vitamin B3,vitamin B6, vitamin C, vitamin E, Folic acid, and mixtures thereof. In this case, the weight ratio in said composition or use is the ratio (vitamin or vitamins of said group) / [(soy derivatives which are not a vitamin of said group) + (polyphenols which are not a vitamin of said group, nor a soy derivative)] is comprised between 1:10 and 10:1, advantageously 1:2 and 5:1.

Preferably, the oral therapeutic formulation comprises at least vitamin B12 and at least another vitamin selected from the group consisting of vitamin B3,vitamin B6, vitamin C, vitamin E, Folic acid, salts thereof and mixtures thereof, whereby the weight ratio vitamin B12/ other vitamins is comprised between 0.0005 and 0.1, preferably between 0.001 and 0.01.

According to a specific embodiment, the oral therapeutic formulation is free of vitamin B3.

Most preferably, the oral therapeutic formulation comprises one or more soy isoflavones as soy derivative.

According to an advantageous embodiment, at least one polyphenol of the oral therapeutic formulation is a polyphenol extracted from grapes (skin and/or fruit and/or juice and/or small branches).
Polyphenol can also be of other origins, such as fruit, seeds, branches, etc. as well as any other parts of plants, such as trees, vegetable, herbs, bushes, leaves, sap, skin, etc. Specific examples of possible origin for polyphenols are : apples, banana, pineapple, blueberry, cantaloupe, cherries, fig, mango, papaya, pear, prickly pear, rasspberry, rhubarb, starfruit, strawberry, watermelon, carrots, chicory, brussels sprouts, tomato, wheat germ, corn, nuts, seeds, spinash, asparagus, potatoes, turnip greens, apricots, pumpkin, cabbage, broccoli, cauliflower, courgette, beans, peas, pimento, celery, cacao plant, tea, tobacco, coffee plant, trees, such as oaks, pine tree, gingko, bark, etc., cannabis, sugar cane, cactus, olive, beech, baldcypress (taxodium), beech (Fagus), birch (betula), boston ivy (Parthennocissus tricuspidata), box tree (Buxus), buddhist pine (podocarpus), cedar (Cedrus), horse-chestnut (Aesculus), chinese quince (Pseudocydonia sinensis), corkbark pine (Pinus thunbergii Parl var. corticosa), crabapple (Malus baccata), crape myrtle (Lagerstroemia indica), current (ribes), dogwood (Cornus dwarf orange), kummaquat (Fortunella hindsii), elm (Ulmus), ezo spruce (Picea glehnii), fir (Abies), fig (ficus), fukien tae (ehretia), flowering quince (Chaenomeles japonica), golden Bellflower (Forsythia), golden larch (pseudolarix), hackberry (Celtis sinensis), haselnut (corylus avellana), hawtorn (craeteagus), holly (Ilex), hornbeam (Carpinus), japanese black pine (Pinus thunbergii), japanese Cedar (Cryptomeria japonica), japanese cypress (Chamaecyparis obtusa), japanese deciduous holly (Ilex serrata), japanese grey black elm (Zelkova serrata), japanese witch hazel (Hamamelis japonica), japanese flowering cherry (Prunus incisa), jessamine (murraya), juniper (Juniperus), larch (Larix), lilac (Syringa vulgaris L. ), linden (tillia), maple (Acer), maidenhair tree (Gingko biloba), oak (Quercus), Oleaster( Elaeagnus), persimmon (Persimmon), pomegranate (Punica Granatum), poplar (populus), privet (Ligustrum), red pine (Pinus ), rosemary (rosmarinus), shrubby cinquefoil (Potentilla fruticosa), snow rose (serissa), spike winterhazel (Corylopsis spicata), spindle tree (Euonymus), spruce(picea), star jasmin (Trachelospermum asiaticum Nakai), sweetgum (liquidambar), trident maple (Acer buergerianum), weeping willow (Salix), white pine (Pinus parviflora), wild Japanese apricot (Prunus mume), winter jasmine (Jasminum nudiflorum), wisteria (Wisteria floribunda), yew (Taxus cuspidata), etc. The plant can possibly genetically modified.

According to a preferred embodiment, the oral therapeutic formulation is free from alcohol, especially ethyl alcohol.

According to a detail of another preferred embodiment, the oral therapeutic formulation further comprises at least a compound selected from the group consisting of EPA, DHA and mixtures thereof.

The invention relates also to a composition for oral administration of at least one vitamin, said composition comprising at least:
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1. The weight ratio in said composition or use is thus the ratio (vitamin or vitamins) / [ (soy derivatives which are not a vitamin) + (polyphenols which are not a vitamin, nor a soy derivative)] is comprised between 1:10 and 10:1, advantageously 1:2 and 5:1.

Advantageously, in said use, the oral therapeutic composition comprises at least vitamin B12 and at least another vitamin selected from the group consisting of vitamin B3,vitamin B6, vitamin C, vitamin E, Folic acid, and mixtures thereof. In this case, the weight ratio in said composition or use is the ratio (vitamin or vitamins of said group) / [ (soy derivatives which are not a vitamin of said group) + (polyphenols which are not a vitamin of said group, nor a soy derivative)] is comprised between 1:10 and 10:1, advantageously 1:2 and 5:1.

Preferably, the oral therapeutic composition comprises at least vitamin B12 and at least another vitamin selected from the group consisting of vitamin B3,vitamin B6, vitamin C, vitamin E, Folic acid, and mixtures thereof, whereby the weight ratio vitamin B12/ other vitamins is comprised between 0.0005 and 0.1, preferably between 0.001 and 0.01.

According to a specific embodiment, the oral therapeutic formulation is free of vitamin B3.

The composition of the invention has advantageously one or more characteristics as defined for the uses of the invention disclosed here above.

According to a preferred embodiment, the composition comprises one or more soy isoflavones as soy derivative.

According to a detail of another preferred embodiment, at least one polyphenol is a polyphenol extracted from grape (fruit, skin, seed, branches, juice, etc.).

The composition is preferably free from ethyl alcohol, but comprises advantageously at least a compound selected from the group consisting of EPA, DHA and mixtures thereof. Fish oil can be used for adding EPA and DHA to the composition.

According to a more specific embodiment, the composition comprises at least soy isoflavones and vitamin B3, the weight ratio vitamin B3/soy isoflavones being greater than 2, preferably comprised between 5 and 25.

According to still a further embodiment, the composition further comprises at least one oligomeric procyanidin, advantageously derived from grape(s) (especially grapes suitable for preparing red wines), whereby the weight ratio soy isoflavones / oligomeric procyanidins is preferably comprised between 5:1 et 1:5.

The composition comprises preferably at least one compound selected from the group consisting of catechins, proantocyanidins,epicatechin, anthocyanins, flavonols and caftaric acid, and possibly further L-arginine.

According to a more specific embodiment, the composition comprises at least vitamin B3, vitamin B6 and vitamin B12, the weight ratio vitamin B3/vitamin B12 being comprised between 10000 and 100,while the weight ratio vitamin B6/vitamin B12 is comprised between 100 and 1. When the composition is free from vitamin B3, the weight ratio vitamin B6/vitamin B12 is preferably comprised between 100 and 1.

According to a specific embodiment, the composition is an oral solid or semi-solid unit dosage form (such as in powder form, pills, tablets, capsules, etc.) comprising less than 1000mg vitamin(s), preferably less than 600mg. for example, said unit dosage form comprises 200mg to 500mg vitamins, 10 to 30 mg soy isoflavones and from 60 mg to 300mg polyphenols.

The composition can further comprises one or more excipients, such as disintegrants, surfactant, filler, acidic compounds, buffer, minerals, amino acids, lipophilic compounds, hydrophilic compounds, bases, tableting agents, etc.

The composition of the invention can be added to a nutriment or to a food, liquid food, pasty food, solid food, such as cereals, milk, soy milk, dry milk, yaourt, cookies, cakes, sweeteners, jelly, candy, pudding, beverage, fruit saps, seasoning compositions, etc. When mixed, the composition of the invention can thus take the form of said food, food additives, beverages, etc.

Details of preferred embodiments will be disclosed hereafter.

### Examples of compositions

Various capsules have been prepared. The composition of said capsules are given in the following table. The used capsules are hypromelose capsules. It is obvious that hard gelatin capsules of soft capsules can be used. It is also obvious that instead of filling a capsule, it is possible to prepare tablets, self disintegrating tablets, pills, liquid formulations, suspensions, etc.

| composition | 1 | 2 | 3 | 4 | 5 |
|---|---|---|---|---|---|
| vitamine B12 | 400µg | 300µg | 200µg | 150µg | 100µg |
| vitamine B3 | 600mg | 500mg | 400mg | 300mg | 200mg |
| vitamine B6 | 24mg | 18mg | 12mg | 10mg | 10mg |
| vitamine C | 200mg | 150mg | 100mg | 100mg | 100mg |
| vitamine E | 120mg | 90mg | 60mg | 50mg | 50mg |
| Folic acid | 500µg | 500µg | 500µg | 500µg | 500µg |
| glycine max (soy) (soy isoflavone content) | 150mg (60mg) | 150mg (60mg) | 120mg (50mg) | 100mg (40mg) | 150mg (60mg) |
| Vitis vinifera fructus (polyphenols content >70% by weight) | 1000mg | 900mg | 600mg | 500mg | 400mg |
| L-arginine | 400mg | 400mg | 300mg | 300mg | 300mg |
| excipient | 200mg | 150mg | 0mg | 100mg | 250mg |

| composition | 6 | 7 | 8 | 9 | 10 |
|---|---|---|---|---|---|
| vitamine B12 | 400µg | 300µg | 200µg | 150µg | 100µg |
| vitamine B3 | 10mg | 5mg | | | |
| vitamine B6 | 24mg | 18mg | 12mg | 10mg | 10mg |
| vitamine C | 200mg | 150mg | 100mg | 100mg | 100mg |
| vitamine E | 120mg | 90mg | 60mg | 50mg | 50mg |
| Folic acid | 500µg | 500µg | 500µg | 500µg | 500µg |
| glycine max (soy) (soy isoflavone content) | 150mg (60mg) | 150mg (60mg) | 120mg (50mg) | 100mg (40mg) | 150mg (60mg) |
| Vitis vinifera fructus (polyphenols content >70% by weight) | 1000mg | 900mg | 600mg | 500mg | 400mg |
| L-arginine | 400mg | 400mg | 300mg | 300mg | 300mg |
| excipient | 200mg | 150mg | 0mg | 100mg | 250mg |

The vitis vinifera fructus contains more than 200 ppm resveratol.
The folic acid can be added in the form of folate, the folate being then expressed in folic acid for determining the dose present in the composition.

Said capsules have been prepared for oral administration to a human with a weight of 70kg. It is advisable to take two capsules a day, a first in the morning and a second at the end of the day. The doses may be slightly different between a day administration and a night administration. For example, the dose will be reduced for a night administration with respect to the dose for a day administration.
The dose to be administered will also depend from the use. For example, when the composition is taken for preventing possible troubles, the doses can be quite low (capsules containing less than 300mg, advantageously less than 200mg of vitamins, polyphenols and soy derivatives)

Instead of making capsules, it is also possible to press said composition, possibly with tabletting agents, matrix, etc. so as to form tablets, matrix, spherules, pellets (such as pellets with a size or diameter of less than 2mm, preferably less than 1mm), etc.

The composition 3 has been tested in a human-like setting animal model for measuring the plasma cholesterol level, the triglyceride level, as well as the liver enzymes. For said tests, the doses to be administered was adapted in function of the weight of the mice. Said test has been carried out on transgenic mice for a four week period. During said period, mice of first group do not received the composition in their hyperlipidaemic diet, while the mice of the second, third and fourth groups receive a hyperlipidaemic diet respectively with 0.045% by weight composition 3, 0.15% by weight composition 3 and 0.45% by weight composition 3. Mice of a further group receive hyperlipidaemic diet with atorvastatin.

### Plasma triglyceride content mmol/l after our weeks administration

| control | diet + 0.045% composition 3 | diet + 0.15% composition 3 | diet + 0.45% composition 3 | diet + atorvastatin |
|---|---|---|---|---|
| about 2.2 | about 1.5 | about 1.2 | about 1.5 | |

The plasma cholesterol LDL content was also reduced when taking diet with 0.045% to 0.15% composition 3

The composition 3 has further been tested in human CRP-transgenic mice for measuring the basal plasma CRP levels and the basal plasma fibrinogen levels.

### basal plasma CRP (µg/ml) after 4 weeks

| control | diet + 0.045% composition 3 | diet + 0.15% composition 3 | diet + 0.45% composition 3 | diet + fenobibrate |
|---|---|---|---|---|
| about 14 | about 8 | about 9 | about 12 | about 3 |

### basal plasma fibrinogen (µg/ml) after 4 weeks

| control | diet + 0.045% composition 3 | diet + 0.15% composition 3 | diet + 0.45% composition 3 | diet + fenofibrate |
|---|---|---|---|---|
| about2.7 | about 2.2 | about 2.2 | about 2.5 | about 1.7 |

This test shows that it is advisable to use reduced doses (0.045% to 0.15%) so as have the best reduction of CRP, fibrinogen, triglyceride and LDL, such a reduction meaning a high reduction of the risk to have cardiovascular diesase.

According to a specific embodiment, the composition with pelletizing agent is extruded and spheronized so as to form pellets with a size of less than 2mm. Said pellets are then placed in pharmaceutically acceptable capsule.

According to another advantageous embodiment, the composition, especially in the form of pellets, comprises a polyglycolyzed glyceride with a HLB greater than 10, preferably greater than 12, such as comprised between 12 and 16. The glyceride has advantageously a melting point greater than 30°C, especially greater than 35°C, such as between 37 and 60°C. A specific example of glyceride is Gelucire ® 44/14 (Gattefossé France).

The composition of the invention (for example the compositions 1 to 10) can be added with one or more further pharmaceutically active agent, such as fenofifibrate, Zocor, etc.

## Claims

1. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for preventing and/or treating cardiovascular diseases.

2. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5 :1,
as therapeutic active mixture for the preparation of an oral therapeutic formulation, especially an oral unit dosage therapeutic form, for reducing the plasma fibrinogen level.

3. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for reducing the blood CRP (C-reactive protein) level.

4. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for preventing or treating atherosclerosis.

5. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for treating or preventing inflammation troubles, preferably vascular inflammation.

6. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for preventing or treating endothelial dysfunction.

7. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, or preventing or treating diabetic vasculitis.

8. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for preventing or treating metabolic syndrome.

9. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for controlling the human CRP gene expression.

10. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5: 1,
as therapeutic active mixture for the preparation of an oral therapeutic formulation, especially an oral unit dosage therapeutic form, for preventing or treating hypertension.

11. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for preventing or treating troubles associated to unstable angina pectoris.

12. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for controlling or reducing the formation of triglycerides.

13. The use of
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1,
as therapeutic active mixture for the preparation of an oral therapeutic formulation, especially an oral unit dosage therapeutic form, for controlling the ratio HDL/LDL and/or for reducing the LDL content.

14. The use according to anyone of the claims 1 to 13, in which the oral therapeutic formulation comprises at least vitamin B12 and at least another vitamin selected from the group consisting of vitamin B3,vitamin B6, vitamin C, vitamin E, Folic acid, and mixtures thereof.

15. The use according to anyone of the claims 1 to 13, in which the oral therapeutic formulation comprises at least vitamin B12 and at least another vitamin selected from the group consisting of vitamin B3,vitamin B6, vitamin C, vitamin E, Folic acid, and mixtures thereof, whereby the weight ratio vitamin B12/ other vitamins is comprised between 0.0005 and 0.1, preferably between 0.001 and 0.01.

16. The use according to anyone of the claims 1 to 13, in which the oral therapeutic formulation comprises one or more soy isoflavones as soy derivative.

17. The use according to anyone of the claims 1 to 13, in which at least one polyphenol of the oral therapeutic formulation is a polyphenol extracted from grape.

18. The use according to anyone of the claims 1 to 13, in which the oral therapeutic formulation is free from ethyl alcohol.

19. The use according to anyone of the claims 1 to 13, in which the oral therapeutic formulation further comprises at least a compound selected from the group consisting of EPA, DHA and mixtures thereof.

20. Composition for oral administration of at least one vitamin, said composition comprising at least :
- at least one vitamin,
- at least one soy derivative which is not a vitamin, and
- at least one polyphenol which is not a vitamin, nor a soy derivative
in which the weight ratio (vitamin or vitamins) / (polyphenol or polyphenols + soy derivative or derivatives) is comprised between 1:10 and 10:1, advantageously between 1:2 and 5:1.

21. The composition of claim 20, which comprises at least vitamin B12 and at least another vitamin selected from the group consisting of vitamin B3,vitamin B6, vitamin C, vitamin E, Folic acid, and mixtures thereof.

22. The composition of claim 20, which comprises at least vitamin B12 and at least another vitamin selected from the group consisting of vitamin B3,vitamin B6, vitamin C, vitamin E, Folic acid, and mixtures thereof, whereby the weight ratio vitamin B12/ other vitamins is comprised between 0.0005 and 0.1, preferably between 0.001 and 0.01.

23. The composition of claim 20, which comprises one or more soy isoflavones as soy derivative.

24. The composition of claim 20, in which at least one polyphenol is a polyphenol extracted from grape.

25. The composition of claim 20, which is free from ethyl alcohol.

26. The composition of claim 20, which further comprises at least a compound selected from the group consisting of EPA, DHA and mixtures thereof.

27. The composition of claim 20, which further comprises fish oil.

28. The composition of claim 20, which comprises at least soy isoflavones and vitamin B3, the weight ratio vitamin B3/soy isoflavones being greater than 2, preferably comprised between 5 and 25.

29. The composition of claim 20, which further comprises at least one oligomeric procyanidin, advantageously derived from red grape(s), whereby the weight ratio soy isoflavones / oligomeric procyanidins is preferably comprised between 5:1 et

30. The composition of claim 20, which comprises at least one compound selected from the group consisting of catechins, proantocyanidins,epicatechin, anthocyanins, flavonol and caftaric acid.

31. The composition of claim 20, which further comprises L-arginine.

32. The composition of claim 20, which comprises at least vitamin B3, vitamin B6 and vitamin B12, the weight ratio vitamin B3/vitamin B12 being comprised between 10000 and 100,while the weight ratio vitamin B6/vitamin B12 is comprised between 100 and 1.

33. The composition of claim 20, as an oral solid or semi-solid unit dosage form comprising less than 1000mg vitamin(s), preferably less than 600mg.

34. the composition of claim 33, in which said unit dosage form comprises 200mg to 500mg vitamins, 10 to 30 mg soy isoflavones and from 60 mg to 300mg polyphenols.
